# EUROPEAN PATENT SPECIFICATION

(11) **EP 4 070 869 B1**
(45) Date of publication and mention of the grant of the patent: **31.01.2024**
(21) Application number: 22174158.0
(22) Date of filing: 29.12.2021
(51) Int. Cl.: G01N 30/88, G01N 30/96, C12N 5/00, B01D 15/34, B01D 15/42, C07K 1/14, C07K 1/16, C07K 14/78

(54) **METHOD OF ANALYZING A MONOMER OF A RECOMBINANT EXTRACELLULAR MATRIX PROTEIN BY SIZE EXCLUSION CHROMATOGRAPHY**
VERFAHREN ZUR ANALYSE EINES MONOMERS EINES REKOMBINANTEN EXTRAZELLULÄREN MATRIXPROTEINS DURCH GRÖSSENAUSSCHLUSSCHROMATOGRAPHIE
MÉTHODE D'ANALYSE D'UN MONOMÈRE D'UNE PROTÉINE DE MATRICE EXTRACELLULAIRE RECOMBINANTE PAR CHROMATOGRAPHIE D'EXCLUSION STÉRIQUE

(30) Priority: 30.12.2020 KR 20200188062
(43) Date of publication of application: 12.10.2022
(62) Divisional of application: 21878746.3
(73) Proprietor: HAPLNSCIENCE INC., Gyeonggi-do 13494 (KR)
(72) Inventor: KIM, Dae Kyong, 13547 Seongnam-si (KR); CHUNG, Yo Kyung, 17098 Yongin-si (KR); HWANG, Sung Mi, 18386 Hwaseong-si (KR)
(74) Representative: Mewburn Ellis LLP

(56) References cited:
- JP-A- 2005 179 240
- JP-A- 2008 024 611
- JP-A- 2017 008 094
- Hazuki Eleanor Miwa: "A recombinant system to model proteoglycan aggregate interactions and aggrecan degradation", CASE WESTERN RESERVE UNIVERSITY, 1 January 2006 (2006-01-01), XP055948956, Retrieved from the Internet: URL:https://etd.ohiolink.edu/apexprod/rws_ etd/send_file/send?accession=case113085783 6&disposition=inline [retrieved on 2022-08-04]
- NOELKEN M E ET AL: "Gel filtration chromatography of triple-helical calf skin collagen", ANALYTICAL BIOCHEMISTRY, ACADEMIC PRESS, AMSTERDAM, NL, vol. 134, no. 2, 15 October 1983 (1983-10-15), pages 374-381, XP024818592, ISSN: 0003-2697, DOI: 10.1016/0003-2697(83)90312-3 [retrieved on 1983-10-15]

## Description

### [Technical Field]

The present disclosure relates to a method of assaying a monomer of a recombinant extracellular matrix protein.

### [Background Art]

The extracellular matrix (ECM) is a non-cellular component in living things, formed by various substances that are secreted out of cells, and is present within all tissues and organs of living organisms. The extracellular matrix performs various biological functions such as cell-to-cell adhesion and physical support, as well as cell differentiation and growth, and intercellular signaling and regulation, etc. Since each tissue and organ of a multicellular organism have independently evolved according to their characteristics, the components and functions of the extracellular matrix also vary depending on the type of tissue and cell.

Basically, the extracellular matrix consists of water, proteins, and polysaccharides. Among them, extracellular matrix proteins are self-assembled into a molecular scaffold by adjusting biomechanical properties and composition according to functions of the corresponding tissue, and most extracellular matrix proteins are expressed in a trace amount in the tissue, and function by forming multiple bonds in a modular form. Due to these structural features, studies regarding mass-production and functions through recombination of extracellular matrix proteins have faced numerous technical challenges. Generally, proteins extracted from animal tissues are used, but the amounts of the extracted proteins are very small. Therefore, to mass-produce extracellular matrix proteins using desired amino acid sequences, a necessity of having more appropriate and cost-effective production systems has been raised.

Meanwhile, it has been reported that hyaluronan and proteoglycan link (HAPLN) protein among extracellular matrix proteins plays a role in stabilizing aggregates of hyaluronic acid and proteoglycan in the extracellular matrix and is involved in cell-to-cell adhesion. There are a total of four types of HAPLN proteins depending on tissues or organs *in vivo* where they are expressed; HAPLN1, HAPLN2, HAPLN3, and HAPLN4. It is known that their functions and roles in each tissue or organ are not substantially distinct.

Korean Patent No. 10-1897340 discloses a pharmaceutical composition for improving skin elasticity or wrinkles, the pharmaceutical composition including HAPLN1 protein as an active ingredient, Korean Patent Publication Nos. 10-2019-0024727 and 10-2020-0104831 disclose a composition for regenerating cartilage and a composition for treating cartilage-related diseases, each composition including HAPLN1 protein as an active ingredient. Recently, Korean Patent No. 10-2166453 discloses a composition for treating lung diseases, the composition including HAPLN1 protein as an active ingredient. As described, HAPLN proteins are expected to provide useful functions to humans. Accordingly, for mass-production of recombinant HAPLN proteins, it is necessary to study culture, isolation, purification, and monomer assay methods. However, a method of mass-producing recombinant HAPLN proteins has not yet been studied.

Miwa, Hazuki E. "A Recombinant System to Model Proteoglycan Aggregate Interactions and Aggrecan Degradation." (2006) relates to a "recombinant system for studies of molecular interactions with hyaluronan (HA), aggrecan, and link protein, as well as for *in vitro* analyses of matrix proteinase-mediated degradation of aggrecan".
JP 2017-008094-A relates to "a collagen-like polypeptide (unimolecule) chain having a higher molecular weight compared to the conventional art". [Disclosure]

### [Technical Problem]

Disclosed herein but not claimed is a medium composition for culturing animal cells for producing a recombinant extracellular matrix protein.

Also disclosed but not claimed is a method of producing the recombinant extracellular matrix protein with high purity.

There is provided a method of assaying a monomer of the recombinant extracellular matrix protein.

### [Technical Solution]

The present invention is as defined in the claims. This and other facets of the present disclosure may be more fully understood by reference to the following description.

Disclosed herein but not claimed is a medium composition for culturing animal cells for producing a recombinant extracellular matrix protein, the medium composition including a copper compound.

The term "extracellular matrix (ECM) protein" refers to a protein present in the extracellular matrix. Exemplary types of the ECM protein include collagen, elastin, fibronectin, laminin, vitronectin, tenacin, hyaluronan and proteoglycan link protein (HAPLN), etc., but are not limited thereto.

The term "hyaluronan and proteoglycan link protein (HAPLN)" is also called hyaluronic acid and proteoglycan link protein. There are a total of four types of HAPLN proteins depending on tissues or organs *in vivo* where they are expressed, and the types thereof include HAPLN1, HAPLN2, HAPLN3, and HAPLN4. An amino acid sequence of the HAPLN protein is described in HAPLN1, for example, human HAPLN1 Accession No. NP_001875, or mouse HAPLN1 Accession No. NP_038528, etc., but is not limited thereto.

The term "recombinant ECM protein" refers to a protein obtained by expressing DNA in cells, the DNA encoding an ECM protein produced using a genetic recombination method. The genetic recombination may be performed according to a common method in the art.

In this disclosure, the recombinant extracellular matrix protein may be collagen, elastin, fibronectin, laminin, vitronectin, tenascin, or HAPLN, but is not limited thereto. In the claimed invention, the recombinant extracellular matrix protein may be elastin, fibronectin, laminin, vitronectin, tenascin, or HAPLN.

The term "recombinant HAPLN protein (rHAPLN)" refers to a protein obtained by inserting a polynucleotide sequence encoding the HAPLN protein into a vector to construct a recombinant vector, introducing the recombinant vector into a host cell, and expressing the recombinant vector in the cell.

The recombinant HAPLN protein may be any one protein selected from the group consisting of HAPLN1, HAPLN2, HAPLN3, and HAPLN4.

The term "recombinant human HAPLN protein (recombinant human HAPLN, rhHAPLN)" refers to a protein obtained by inserting a polynucleotide sequence encoding the human HAPLN protein into a vector to construct a recombinant vector, introducing the recombinant vector into a host cell, and expressing the recombinant vector in the cell.

The recombinant extracellular matrix protein may be a protein derived from a human body or an animal. The recombinant extracellular matrix protein may be a protein derived from a human body. The term "animal cells for producing the recombinant ECM protein" or "animal cells producing the recombinant ECM protein" refers to animal cells into which the recombinant vector is introduced to produce the recombinant ECM protein.

The animal cells are not limited to the type thereof, as long as they are cells capable of producing the recombinant ECM protein. The animal cells may be selected from the group consisting of Chinese Hamster Ovary (CHO), VERO, Baby Hamster Kidney (BHK), HeLa, NiH 3T3, Madin-Darby Canine Kidney (MDCK), WI38, Human Embryonic Kidney (HEK), hybridoma, and NSO cells. The animal cells may be CHO cells or CHO cell variants. The CHO cells may be CHO-K1, CHO-DXB11, CHO-DG44, CHO-S, or CHO-Pro minus cells.

The term "copper compound" refers to a compound of copper, and copper with the oxidation state of +1, +2, or +3 is known.

The type of the copper compound included in the medium composition disclosed herein is not limited. The copper compound may be a copper (I) compound, a copper (II) compound, or a copper (III) compound. The copper compound may be copper oxide (Cu₂O), copper chloride (CuCl₂), copper nitrate (Cu(NO₃)₂), copper II oxide (CuO), copper sulfide (CuS), or copper sulfate (CuSO₄), but is not limited thereto.

The medium composition may include the copper compound at a concentration of more than about 20 µM, about 30 µM or more, about 40 µM or more, about 50 µM or more, for example, more than about 20 µM to about 1,000 µM, about 30 µM to about 1,000 µM, about 40 µM to about 1,000 µM, about 50 µM to about 1,000 µM, about 50 µM to about 900 µM, about 50 µM to about 800 µM, about 50 µM to about 700 µM, about 50 µM to about 600 µM, about 50 µM to about 500 µM, about 50 µM to about 400 µM, about 50 µM to about 300 µM, about 50 µM to about 200 µM, about 50 µM to about 100 µM, about 50 µM to about 90 µM, about 50 µM to about 80 µM, about 50 µM to about 70 µM, about 50 µM to about 60 µM, or about 50 µM. The medium composition may separate, into monomers, the ECM protein that binds with multiple bonds in a modular form by including the copper compound at a concentration of more than about 20 µM, particularly, about 50 µM or more. When the concentration of the copper compound is about 20 µM or less, formation of protein multimers may be increased, and thus a protein production amount may be decreased. Therefore, by including the copper compound at a concentration of more than about 20 µM, particularly, about 50 µM or more, the medium composition may reduce formation of recombinant ECM protein multimers. Consequently, when the medium composition is used, the ECM protein may be separated into monomers, and thus it is possible to solve the problem of difficulty in mass-production due to the structural feature in which the ECM protein is combined in a modular form after being expressed at a trace amount.

The medium composition may further include general medium components used for culturing animal cells. The general medium components are components needed for culturing animal cells to obtain the recombinant protein, and any known or commercially available components may be used.

The medium composition may further include an additional additive. The medium composition may further include one or more additives of dimethyl sulfoxide (DMSO); glycerol; poloxamers such as poloxamer 188; EDTA; polysorbates such as polysorbate 80; cysteine; glutathione (GSH); glutathione disulfide (GSSG); and magnesium chloride (MgCl₂), but is not limited thereto.

It was confirmed that when copper sulfate was added to a medium during culturing animal cells producing a recombinant human HAPLN1 protein, it exhibited the excellent effect of reducing formation of protein multimers, and thus the protein production amount was increased. Therefore, the medium composition including the copper compound may be usefully applied to obtaining a large amount of the recombinant ECM protein from animal cells.
the disclosed medium composition may be a medium composition for culturing animal cells for mass-producing the recombinant HAPLN protein, specifically, a medium composition for culturing animal cells for mass-producing the recombinant human HAPLN protein, and more specifically, a medium composition for culturing animal cells for mass-producing the recombinant human HAPLN1 protein.

In the medium composition, the recombinant extracellular matrix protein may be a monomer. The "monomer" is used interchangeably with a "monomer protein", and refers to one of proteins constituting a multi-protein complex. A complex of two or more polypeptides is referred to as a "multimer" or an "oligomer", a complex of two polypeptides is referred to as a dimer, a complex of three polypeptides is referred to as a trimer, and a complex of four polypeptides is referred to as a tetramer. Since the medium composition including the copper compound has the effect of reducing formation of the multimers of the recombinant ECM protein, it may be used for producing the monomer of the recombinant ECM protein. In other words, the medium composition including the copper compound may separate, into monomers, the ECM protein that binds with multiple bonds in a modular form. Therefore, the medium composition may be a medium composition for culturing animal cells for producing the monomer of the recombinant ECM protein.

Also disclosed herein but not claimed is a method of producing the recombinant extracellular matrix protein with high purity, the method including
(1) culturing animal cells producing the recombinant ECM protein in the medium composition according to the disclosure and obtaining a culture medium; and
(2) isolating and purifying the recombinant ECM protein from the culture medium.

The medium composition, the recombinant ECM protein, and the animal cells are the same as described above.

According to the method of producing the recombinant ECM protein, the recombinant ECM protein with high purity may be mass-produced. Therefore, the method may be a method of mass-producing the recombinant ECM protein, specifically, a method of mass-producing the recombinant HAPLN protein, more specifically, a method of mass-producing the recombinant HAPLN1 protein, and most specifically, a method of mass-producing the recombinant human HAPLN1 protein.

The culturing of (1) may be performed using a method widely known in the art. For example, the culturing may be performed by fed-batch culture, continuous culture, batch culture, etc. In some cases, the culturing of (1) may be fed-batch culture.

The term "fed-batch culture", which is a culture method of intermittently feeding a medium, refers to a culture method capable of freely controlling an amount of a substrate to be fed, because the substrate in the culture medium is added at an appropriate rate without discharge.

The term "continuous culture" refers to a culture method, in which a new nutrient medium is continuously fed, and at the same time, the culture medium containing cells and products is continuously removed.

The term "batch culture", which is a method of continuing culture until all of raw material substrates initially fed are consumed, refers to a culture method, in which concentrations of a substrate, metabolites, and cells are continuously changed over time.

The culturing of (1) may be performed for about 5 days to about 15 days, about 5 days to about 13 days, about 8 days to about 15 days, about 8 days to about 13 days, about 10 days to about 15 days, about 10 days to about 13 days, about 11 days to about 15 days, or about 11 days to about 13 days, but is not limited thereto.

In (1), the copper compound may be added once or twice or more to the medium composition during cell culture.

In (1), the culturing may be performed by adding the copper compound to the medium composition before cell culture. For example, the copper compound may be added to the medium composition on day 0 of cell culture.

In the method of producing the recombinant ECM protein, the recombinant ECM protein may be a monomer. Therefore, the method may be a method of producing a monomer of the recombinant ECM protein, specifically, a method of producing a monomer of the recombinant HAPLN protein, more specifically, a method of producing a monomer of the recombinant HAPLN1 protein, and most specifically, a method of producing a monomer of the recombinant human HAPLN1 protein.

(2) may include performing chromatography. The chromatography may be any one or more selected from the group consisting of affinity chromatography, anion exchange chromatography, cation exchange chromatography, hydroxyapatite chromatography, reversed-phase chromatography, size exclusion chromatography, mixed mode chromatography, and hydrophobic interaction chromatography.

(2) may include performing anion exchange chromatography.

The term "ion exchange chromatography (IEC)" refers to a method of separating and analyzing sample ions using an affinity difference for a stationary phase by performing a reversible ion exchange between the stationary phase and a mobile phase using an ion exchanger in the stationary phase.

The term "anion exchange chromatography (AEX)" is a type of ion exchange chromatography, and uses an anion exchanger having a cationic functional group such as an amino group, etc.

The anion exchange chromatography may include pre-equilibration, equilibration, sample loading, washing, and elution.

The anion exchange chromatography may be performed using a common anion exchange resin. Examples of the anion exchange resin may include Fractogel^{®} EMD TMAE (M), Fractogel^{®} EMD TMAE Medcap (M), Fractogel^{®} EMDTMAEHicap (M), Eshmuno^{®} Q, Eshmuno^{®} QPX, Eshmuno^{®} QPX Hi cap, Capto Q, Capto Q ImpRes, Q Sepharose^{®} FF, Q Sepharose^{®} HP, Q Sepharose^{®} XL, Source^{®} 30Q, Capto^{®} Adhere, Capto^{®} Adhere ImpRes, Poros^{®} 50 HQ, Poros^{®} 50 XQ, Poros^{®} 50 PI, Q HyperCel, Toyopearl^{®} GigaCap Q 650-M, Toyopearl^{®} GigaCap Q 650-S, Toyopearl^{®} Super Q, YMC^{®} BioPro Q, Macro-Prep^{®} High Q, Nuvia^{®} Q, UNOsphere^{®} Q, etc., but are not limited thereto. Alternatively, a weak anion exchange resin with diethylaminoethyl (DEAE) of dimethylaminoethyl (DMAE) functional group may also be used, depending on operating conditions and pI of the protein. Examples thereof include Fractogel^{®} EMD DEAE, Fractogel^{®} EMD DMAE, Capto^{®} DEAE or DEAE Ceramic HyperD^{®} F.

The anion exchange chromatography may be performed in a bind-and-elute mode, but is not limited thereto.

A loading amount of the anion exchange chromatography may be 10 g/L to 50 g/L resin, but is not limited thereto.

An elution buffer of the anion exchange chromatography may include histidine hydrochloride (His-HCl).

The elution buffer of the anion exchange chromatography may include about 1 mM to about 1000 mM, about 10 mM to about 800 mM, about 20 mM to about 600 mM, about 40 mM to about 400 mM, about 60 mM to about 200 mM, for example, about 100 mM of histidine hydrochloride (His-HCl). When histidine hydrochloride is used in the above concentration range, the recombinant ECM protein may be isolated with excellent purity and yield.

The elution buffer of the anion exchange chromatography may further include EDTA. A concentration of EDTA may be appropriately selected by those skilled in the art.

The elution buffer of the anion exchange chromatography may be at pH 4.0 to pH 6.0, at pH 4.5 to pH 5.5, for example, at pH 5.0, but is not limited thereto.

The recombinant ECM protein may be captured by performing the anion exchange chromatography. Therefore, a specific recombinant ECM protein may be specifically isolated by performing the anion exchange chromatography.

(2) may further include performing cation exchange chromatography after performing the anion exchange chromatography.

The term "cation exchange chromatography (CEX)" is a type of ion exchange chromatography, and uses a cation exchanger having an anionic functional group such as a sulfone group, a carboxyl group, etc.

The cation exchange chromatography may include equilibration, sample loading, washing I, washing II, washing III, and elution.

The cation exchange chromatography may be performed using a common cation exchange resin. Examples of the cation exchange resin may include Eshmuno^{®} CPS, Eshmuno^{®} CPX, or SP Fast Flow Sepharose^{®}, Eshmuno^{®} S Resin, Fractogel^{®} SO3(M), Fractogel SE Hicap (M), SP Cellthru BigBead Plus^{®} , Streamline^{®} SP, Streamline^{®} SP XL, SP Sepharose^{®} Big Beads, Toyopearl^{®} M-Cap II SP-550EC, SP Sephadex^{®} A-25, Express-Ion^{®} S, Toyopearl^{®} SP-550C, Toyopearl^{®} SP-650C, Source^{®} 30S, Poros^{®} 50 HS, Poros^{®} 50 XS, SP Sepharose^{®} Fast Flow, SP Sepharose^{®} XL, Capto^{®} S, Capto^{®} SP ImRes, Capto^{®} S ImpAct, Nuvia^{®} HR-S ,Cellufine^{®} MAX S-r, Cellufine^{®} MAX S-h, Nuvia^{®} S, UNOsphere^{®} S, UNOsphere^{®} Rapid S, Toyopearl^{®} Giga-Cap S-650 (M), S HyperCel Sorbent^{®}, Toyopearl^{®} SP-650M, Macro-Prep^{®} High S, Macro-Prep^{®} CM, S Ceramic HyperD^{®} F, MacroCap^{®} SP, Capto^{®} SP ImpRes, Toyopearl^{®} SP-650S, SP Sepharose^{®} High Perform, Capto^{®} MMC, Capto^{®} MMC Imp Res, Eshmuno^{®} HCX, Nuvia^{®} High c-Prime, etc., but are not limited thereto. Alternatively, a weak cation exchange resin, for example, Fractogel^{®} EMD COO (M), CM Sepharose^{®} HP, CM Sepharose^{®} FF, Toyopearl^{®} AF Carboxy 650-M, Macro-Prep^{®} CM, Toyopearl^{®} GigaCap CM, CM Ceramic Hyper^{®} D, or Bio-Rex^{®} 70 may also be used, depending on operating conditions and pI of the protein.

The cat ion exchange chromatography may be performed in a bind-and-elute mode, but is not limited thereto.

A loading amount of the cation exchange chromatography may be 10 g/L to 50 g/L resin, but is not limited thereto.

In the washing II of the cation exchange chromatography, a washing buffer II may include about 1 mM to about 1000 mM, about 5 mM to about 800 mM, about 10 mM to about 400 mM, about 25 mM to about 200 mM, or about 50 mM to about 150 mM, for example, about 100 mM of sodium chloride (NaCl).

In the washing III of the cation exchange chromatography, a washing buffer III may include about 150 mM to about 500 mM, about 150 mM to about 400 mM, about 200 mM to about 500 mM, about 200 mM to about 400 mM, about 300 mM to about 500 mM, or about 300 mM to about 400 mM, for example, about 350 mM of sodium chloride (NaCl).

The washing buffer II or III of the cation exchange chromatography may further include Tris-HCl, NaAc, EDTA, or a combination thereof.

The washing buffer II or III of the cation exchange chromatography may be at pH 5.0 to 8.5, for example, at pH 8.0 or pH 5.5, but is not limited thereto.

An elution buffer of the cation exchange chromatography may include about 50 mM to about 1000 mM, about 100 mM to about 800 mM, about 200 mM to about 600 mM, about 300 mM to about 500 mM, for example about, 370 mM of sodium chloride (NaCl). A concentration of sodium chloride may be appropriately selected in consideration of a balance between purity and yield of the product within the above range.

The elution buffer of the cation exchange chromatography may further include Tris-HCl, EDTA, or a combination thereof.

The elution buffer of the cation exchange chromatography may be at pH 7.5 to pH 8.5, for example, at pH 8.0, but is not limited thereto.

By performing the cation exchange chromatography, protein aggregates, host cell proteins (HCPs), and other impurities may be removed.

The term "aggregates" refers to a form in which several substances are combined together. The "protein aggregates" refers to a form in which proteins are accumulated or assembled, and includes aggregates of abnormal proteins as well as aggregates of normal proteins. The protein aggregates include those in which target proteins are bound together with other proteins, but there is a difference in that multimeric proteins refer to those in which target proteins are bound to each other.

The term "host cell proteins (HCPs)" refers to process-related protein impurities produced by a host organism during preparation and production of biotherapeutic agents.

(2) may further include performing mixed mode chromatography after performing the cation exchange chromatography.

The term "mixed-mode chromatography (MMC)" refers to a chromatographic method utilizing one or more types of interactions between a stationary phase and an analyte.

The mixed mode chromatography may include pre-equilibrat ion, equilibration, sample loading, washing I, washing II, and elution.

The mixed mode chromatography may be performed using a common mixed mode resin. Examples of the mixed mode resin may include Capto ^{®} adhere, etc., but are not limited thereto.

The mixed mode chromatography may be performed in a bind-and-elute mode, but is not limited thereto.

A loading amount of the mixed mode chromatography may be 10 g/L resin to 15 g/L resin, but is not limited thereto.

In the washing II of the mixed mode chromatography, a washing buffer II may include about 200 mM to about 400 mM, about 200 mM to about 350 mM, or about 250 mM to about 350 mM, for example, about 200 mM or about 300 mM of arginine.

The washing buffer II of the mixed mode chromatography may further include Tris-HCl, EDTA, or a combination thereof.

The washing buffer II of the mixed mode chromatography may be at pH 8.5 to 9.5, for example, at pH 9.0, but is not limited thereto.

The elution buffer of the mixed mode chromatography may include about 100 mM to about 1000 mM, about 200 mM to about 800 mM, about 300 mM to about 700 mM, about 400 mM to about 600 mM, for example, about 500 mM of arginine. A concentration of arginine may be appropriately selected in consideration of a balance between purity and yield of the product within the above range.

The elution buffer of the mixed mode chromatography may further include Tris-HCl, EDTA, or a combination thereof.

The elution buffer of the mixed mode chromatography may be at pH 7.5 to pH 8.5, for example, at pH 8.0, but is not limited thereto.

By performing the mixed mode chromatography, protein aggregates and HCPs may be removed.

(2) may further include performing hydrophobic interaction chromatography after performing the mixed mode chromatography.

The term "hydrophobic interaction chromatography (HIC)" refers to a chromatographic method utilizing hydrophobic interactions between a functional group of a stationary phase and an analyte.

The hydrophobic interaction chromatography may include equilibration, sample loading, washing I, washing II, washing III, and elution.

The hydrophobic interaction chromatography may be performed using a common hydrophobic interaction resin. Examples of the hydrophobic interaction resin may include Butyl-S Sepharose 6 Fast Flow, Capto Octyl, Octyl Sepharose 4 Fast Flow, Phenyl Sepharose 6 Fast Flow (low sub), Capto Butyl, Butyl Sepharose 4 Fast Flow, Phenyl Sepharose High Performance, Capto Phenyl ImpRes, Butyl Sepharose High Performance, Capto Butyl ImpRes, Phenyl Sepharose 6 Fast Flow (high sub), Capto Phenyl (high sub), etc., but are not limited thereto.

The hydrophobic interaction chromatography may be performed in a bind-and-elute mode, but is not limited thereto.

A loading amount of the hydrophobic interaction chromatography may be 3 g/L to 6 g/L resin.

In the washing II of the hydrophobic interaction chromatography, a washing buffer II may include about 0.1 M to about 1.0 M, about 0.1 M to about 0.8 M, about 0.1 M to about 0.6 M, about 0.1 M to about 0.5 M, about 0.2 M to about 1.0 M, about 0.2 M to about 0.8 M, about 0.2 M to about 0.6 M, about 0.2 M to about 0.4 M, about 0.3 M to about 1.0 M, about 0.3 M to about 0.8 M, about 0.3 M to about 0.6 M, or about 0.3 M to about 0.5 M, for example, about 0.4 M of ammonium sulfate.

The washing buffer II of the hydrophobic interaction chromatography may further include Tris-HCl, EDTA, or a combination thereof.

The washing buffer II of the hydrophobic interaction chromatography may be at pH 7.5 to pH 8.5, for example, at pH 8.0, but is not limited thereto.

In the washing III of the hydrophobic interaction chromatography, a washing buffer III may include about 0.5 M to about 2.0 M, about 0.5 M to about 1.8 M, about 1.0 M to about 2.0 M, about 1.0 M to about 1.8 M, about 1.2 M to about 2.0 M, or about 1.2 M to about 1.8 M, for example, about 1.5 M of sodium chloride.

The washing buffer III of the hydrophobic interaction chromatography may further include Tris-HCl, EDTA, or a combination thereof.

The washing buffer III of the hydrophobic interaction chromatography may be at pH 7.5 to pH 8.5, for example, at pH 8.0, but is not limited thereto.

An elution buffer of the hydrophobic interaction chromatography may include about 0.1 M to about 1.5 M, about 0.1 M to about 1.2 M, about 0.1 M to about 1.0 M, about 0.1 M to about 0.8 M, about 0.3 M to about 1.5 M, about 0.3 M to about 1.2 M, about 0.3 M to about 1.0 M, or about 0.3 M to about 0.8 M, for example, about 0.5 M of sodium chloride (NaCl). When the concentration of sodium chloride exceeds 1.5 M, the recombinant ECM protein may not be eluted.

The elution buffer of the hydrophobic interaction chromatography may further include Tris-HCl.

The elution buffer of the hydrophobic interaction chromatography may be at pH 7.5 to 8.5, for example, at pH 8.0, but is not limited thereto.

By performing the hydrophobic interaction chromatography, protein multimers and HCPs may be removed.

(2) may sequentially include performing anion exchange chromatography; performing cation exchange chromatography; performing mixed mode chromatography; and performing hydrophobic interaction chromatography. (2) may further include a known method capable of isolating and purifying the recombinant protein. For example, harvest and clarification, ultrafiltration, diafiltration, solvent/detergent (S/D) virus inactivation, intermediate depth filtration, or a combination of two or more thereof may be further performed. The harvest and clarification, ultrafiltration, diafiltration, S/D virus inactivation, and intermediate depth filtration may be performed according to common methods.

(2) may sequentially include performing harvest and clarification of the culture medium obtained in (1); performing ultrafiltration and diafiltration; performing anion exchange chromatography; performing S/D virus inactivation: performing cation exchange chromatography; performing mixed mode chromatography; performing hydrophobic interaction chromatography; performing ultrafiltration and diafiltration; and performing intermediate depth filtration.

The claimed invention provides a method of assaying the monomer of the recombinant ECM protein, the method including
performing size exclusion chromatography on a sample including the recombinant ECM protein using a mobile phase containing hydrochloride; and
analyzing the monomer of the recombinant ECM protein in the sample, based on the result of the size exclusion chromatography. In the claimed invention, the recombinant extracellular matrix protein may be elastin, fibronectin, laminin, vitronectin, tenascin, or hyaluronan and proteoglycan link protein (HAPLN).

The method of assaying the monomer of the recombinant ECM protein may be a method of assaying the monomer of the recombinant HAPLN protein, more specifically, a method of assaying the monomer of the recombinant HAPLN1 protein, and most specifically, a method of assaying the monomer of the recombinant human HAPLN1 protein.

When hydrochloride is included in the mobile phase, the ability to isolate the recombinant ECM protein may be increased and inaccurate peaks in the chromatogram may be remarkably reduced.

The hydrochloride included in the mobile phase is not limited to its type. The hydrochloride may be arginine hydrochloride (Arg-HCl), aniline hydrochloride, adenine hydrochloride, guanine hydrochloride, guanidine hydrochloride (Gdn-HCl), histidine hydrochloride (His-HCl), or lysine hydrochloride (Lys-HCl), but is not limited thereto.

In the claimed invention, the mobile phase includes the hydrochloride at a concentration of 0.5M to 1.2M. In other disclosed but not necessarily claimed cases, the mobile phase may include the hydrochloride at a concentration of more than about 0.5 M, about 0.8 M or more, about 1.0 M or more, more than about 0.5 M to about 10.0 M, more than about 0.5 M to about 8.0 M, more than about 0.5 M to about 4.0 M, about 0.8 M to about 4.0 M, about 0.8 M to about 3.0 M, about 0.8 M to about 2.0 M, about 0.8 M to about 1.5 M, about 0.8 M to about 1.2 M, about 1.0 M or more to about 10.0 M, about 1.0 M or more to about 8.0 M, about 1.0 M or more to about 4.0 M, about 1.0 M or more to about 3.0 M, or about 1.0 M to about 2.0 M When the concentration of hydrochloride is 0.5 M or less, the ability to isolate the recombinant ECM protein may be decreased. When the concentration of hydrochloride is 0.5 M or less, the ability to assay the monomer of the recombinant ECM protein may be decreased.It was confirmed that when hydrochloride is used as an additive for the mobile phase, the monomer of the recombinant ECM protein may be accurately analyzed even at a low concentration of about 1.0 M.

The term "size exclusion chromatography (SEC)", which is also referred to as "gel filtration chromatography", is a method of separating proteins according to their size. There is no attractive force between a stationary phase and a solute, and a mobile phase simply passes through the porous stationary phase, unlike other types of chromatography.

The size exclusion chromatography may be performed according to a common method. The size exclusion chromatography may be size exclusion chromatography for analysis.

Since the method assays the monomer of the recombinant ECM protein, it may accurately analyze ratios of the monomer of the recombinant ECM protein and other impurities (e.g. , multimers, etc.) in the products after performing each isolation and/or purification during the process of producing the recombinant ECM protein. Accordingly, the ratio of the monomer of the recombinant ECM protein may be analyzed. Therefore, in the analysis, it is possible to analyze the ratios of the monomer of the recombinant ECM protein and other impurities.

The method of assaying the monomer of the recombinant ECM protein may analyze a ratio of the monomer of the recombinant HAPLN protein, more specifically, a ratio of the monomer of the recombinant HAPLN1 protein, and most specifically, a ratio of the monomer of the recombinant human HAPLN1 protein.

### [Advantageous Effects]

According to a disclosed but not claimed medium composition according to the disclosure, animal cells producing a recombinant ECM protein may be cultured in a large amount.

According to a disclosed but not claimed method of producing a recombinant ECM protein according to the disclosure, the recombinant ECM protein may be isolated with high purity and a monomer of a specific recombinant ECM protein may be specifically isolated.

According to a method of assaying a monomer of the recombinant ECM protein according to the claimed invention, the monomer of the recombinant ECM protein may be analyzed with high accuracy, and thus ratios of the monomer of the recombinant ECM protein and other impurities may be analyzed.

### [Description of Drawings]

FIG. 1 shows a chromatogram of a linear gradient elution of anion exchange chromatography (AEX);
FIG. 2 shows a chromatogram of a step-wise elution of cation exchange chromatography (CEX);
FIG. 3 shows a result of SDS_PAGE_NR of the step-wise elution of CEX;
FIG. 4 shows a chromatogram of a step-wise elution of mixed-mode chromatography (MMC);
FIG. 5 shows a result of SDS_PAGE_NR of the step-wise elution of MMC;
FIGS. 6A and 6B show a chromatogram of hydrophobic interaction chromatography (HIC) comparative elution;
FIG. 7 shows a result of SDS_PAGE_NR of HIC comparative elution;
FIG. 8 shows a chromatogram showing a result of performing size exclusion chromatography (SEC) analysis for a sample including rhHAPLN1 using phosphate buffer(PB)+NaCl, 5 mM EDTA, or 5 mM EDTA+4 M Gdn-HCl as a mobile phase;
FIG. 9 shows a chromatogram showing a result of performing SEC analysis for a sample including rhHAPLN1 using 50 mM PB+150 mM NaCl+1M Arg-HCl (pH 6.3) as a mobile phase;
FIG. 10 shows a chromatogram showing a result of performing SEC analysis for Sample 3 using 50 mM PB+300 mM NaCl, 0.1 MArg-HCl, 0.5 M Arg-HCl, 1.0 MArg-HCl, or 1.0 M Gdn-HCl as a mobile phase;
FIG. 11 shows a chromatogram showing a result of performing SEC analysis for Sample 3 using 50 mM PB+300 mM NaCl, 0.1 M urea, 0.5 M urea, 1.0 M urea, 2.0 M urea, 4.0 M urea, or 6.0 M urea as a mobile phase; and
FIG. 12 shows a chromatogram showing a result of performing SEC analysis for Sample 3 using 1.0 M Gdn-HCl, 4.0 M urea, or 1.0 M Arg-HCl as a mobile phase.

Hereinafter, the present disclosure will be described in more detail with reference to exemplary embodiments.

### Example 1 (not according to the invention): Culture of Cells Producing Recombinant ECM Protein

A vector including a polynucleotide encoding a human HAPLN1 protein among ECM proteins was inserted to CH0-K1 cells to prepare a CH0-K1 cell line producing the recombinant human HAPLN1 protein. A cell line showing excellent protein production amount and quality was selected as a master cell bank (MCB).

The MCB was sub-cultured and inoculated at a concentration of 0.40±0.05×10⁶ cells/mL in a 250 L Hyperforma SUB bioreactor (Thermo), followed by fed-batch culture.

22.36 g of Act iPro^{™} medium + 0.5846 g of glutamine + 10.00 g of HT Supplement (Thermo Fisher Scientific) + 4.29 g of 10 N NaOH + 1.80 g of NaHCO₃ were used as a basic medium. A culture temperature, dissolved oxygen (DO), and pH were set to 36.5 °C, 40.0%, and 7.00 ± 0.20, respectively. 1 M sodium carbonate monohydrate was used as a pH control solution.

181.04 g of HyClone^{™} Cell Boost 7a + 12.28 g of FM020a of 10 N NaOH, and 94.60 g of HyClone^{™} Cell Boost 7b + 105.93 g of FM020b of 10 N NaOH were used as a feeding medium (FM). A feeding strategy is shown as in Table 1 below.

**[Table 1]**

| | Fed-batch culture | | | | |
|---|---|---|---|---|---|
| Feeding medium | Day 3 | Day 5 | Day 6 | Day 8 | Day 10 |
| FM020a (%(w/w)) | 3 | 5 | 3 | 3 | 3 |
| FM020b (%(w/w)) | 0.3 | 0.5 | 0.3 | 0.3 | 0.3 |

As a feed additive, 50 µM CuSO₄ was used, which was added to the bioreactor on day 0 of fed-batch culture.

A Glucose feed stock was prepared at 400 g glucose/kg. From day 3 to day 13 of fed-batch culture, when the glucose concentration dropped to less than 5.0 g/L, glucose was fed in such a way that it was raised to 6.0 g/L.

When a viable cell density (VCD) reached 20.00×10⁶ cells/mL, the temperature was changed to 31.0 °C. Cells were harvested on 12 day of fed-batch culture or when viability dropped to less than 60%.

### Experimental Example 1 (not according to the invention): Effect of Reducing Formation of Multimer of Recombinant ECM Protein according to Type of Additive

An experiment was performed to examine an effect of reducing formation of protein multimers according to the type of additive when cells producing the recombinant human HAPLN1 protein were cultured.

In detail, cells were cultured in the same manner as in Example 1, except for varying the type, concentration, and feeding strategy of the feeding additive. Results of cell culture and protein production according to the type and concentration of the feeding additive, and feeding strategy thereof in each experimental group are shown in Table 2 below.

**[Table 2]**

| Experimental group | Type of additive* | Concentration of additive | Feeding strategy | Titer (g/L) | Qp (pg/cell/day) | SEC_LC (Main)(%) |
|---|---|---|---|---|---|---|
| Comparative Example 1 | - | - | - | 2.66 | 21.08 | 51.1 |
| Comparative Example 2 | DMSO | 0.5%(w/v) | Feeding on days 4, 6, 8 | 2.22 | 18.01 | 44.8 |
| Comparative Example 3 | DMSO | 0.2%(w/v) | Feeding on days 0, 3, 5, 7, 9 | 2.42 | 19.15 | 44.4 |
| Comparative Example 4 | Glycerol | 1.0%(w/v) | Feeding on day 5 | 2.46 | 20.46 | 43.5 |
| Comparative Example 5 | Poloxamer 188 | 0.2%(w/v) | Feeding on day 5 | 2.71 | 22.49 | 47.0 |
| Comparative Example 6 | EDTA | 1 mM | Feeding on day 5 | 2.03 | 20.96 | 57.5 |
| Comparative Example 7 | PS80 | 0.04%(w/v) | Feeding on day 5 | 2.75 | 23.77 | 43.8 |
| Comparative Example 8 | Cysteine | 1 mM | Feeding on days 5, 7, 9 | 1.91 | 15.83 | 31.4 |
| Comparative Example 9 | GSH | 0.2 mM | Feeding on days 6, 8 | 2.36 | 19.48 | 43.5 |
| Comparative Example 10 | GSH and GSSG | 0.2 mM and 0.2 mM | Feeding on days 6, 8 | 2.52 | 20.31 | 47.0 |
| Comparative Example 11 | EDTA and MgCl₂ | 1 mM and 50 µM | Feeding on day 5 | 2.16 | 22.34 | - |
| Example 1 | CuSO₄ | 50 µM | Feeding on day 0 | 3.87 | 28.77 | 53.2 |

| | | | | | | |
|---|---|---|---|---|---|---|
| *DMSO: dimethyl sulfoxide, PS80: polysorbate 80, GSH: glutathione, GSSG: glutathione disulfide. | | | | | | |

As shown in Table 2, when CuSO₄ additive was used, a titer of the recombinant human HAPLN1 protein production was 3.87 g/L, which was the highest, and the production amount of the recombinant human HAPLN1 protein per cell was 28.77 pg/cell/day, which was the highest.

Therefore, it was confirmed that when a copper compound such as CuSO₄ is used as an additive during culturing cells producing the recombinant human HAPLN1 protein, the effect of reducing formation of the protein multimer is excellent and the protein production amount is increased. Specifically, when CuSO₄ at a concentration of 50 µM was fed on day 0 of cell fed-batch culture, the highest effect of reducing formation of the protein multimer and the highest protein production amount were observed. Accordingly, it is suggested that may be used in mass-production of the recombinant human HAPLN1 protein.

### Experimental Example 2 (not according to the invention): Effect of Reducing Formation of Recombinant ECM protein Multimer according to Concentration of Copper Compound

An experiment was performed to examine an effect of reducing formation of protein multimers according to concentrations of the copper compound when cells producing the recombinant human HAPLN1 protein were cultured.

In detail, cells were cultured in the same manner as in Example 1, except for varying the concentration of the copper compound. The concentration of the copper compound in each experimental group and results of protein production according to the concentration are shown in Table 3 below.

**[Table 3]**

| Experiment al group | CuSO₄ concentr at ion | Titer (D10) (g/L) | SEC | | | Caliper_NR | | | |
|---|---|---|---|---|---|---|---|---|---|
| | | | Main Peak % | HMW Peak % | LMW Peak % | Pur i ty % | peak1 % | Peak2% | Peak3% |
| Example 1 | 50 µM | 2.57 | 50.3 | 38.5 | 11.2 | 85.7 | 20.6 | 65.1 | 0.68 |
| Comparativ e Example 12 | 20 µM | 2.42 | 51.8 | 38.8 | 9.4 | 86.7 | 20.9 | 65.8 | 0.67 |
| Comparativ e Example 13 | 5 µM | 2.42 | 51.3 | 38.8 | 9.9 | 86.0 | 20.6 | 65.4 | 0.71 |

As shown in Table 3, when CuSO₄ was used at a concentration of more than 20 µM, a titer of the human HAPLN1 protein product ion was increased. In particular, Example 1, in which CuSO₄ was used at a concentration of 50 µM or more, showed the excellent titer of the human HAPLN1 protein production. Therefore, when a copper compound such as CuSO₄ is used at a concentration of more than 20 µM, particularly, 50 µM or more during culturing the cells producing the recombinant human HAPLN1 protein, the excellent effect of reducing formation of the protein multimer and the increased protein production amount were observed.

### Example 2 (not according to the invention): Isolation and Purification of Recombinant ECM Protein

The recombinant human HAPLN1 protein was isolated and purified from the cells cultured according to Example 1. In detail, isolation and purification of the recombinant human HAPLN1 protein were performed according to the following procedures.

### (1) Harvest and Clarification

For harvest and clarification, DOHC and A1HC depth filter of Millipore were used. Recommended loading volumes of DOHC and A1HC depth filters are 45 L/m² and 90 L/m², respectively.

### (2) Ultrafiltration/Diafiltration 1 (UF/DF1)

Pellicon 3 (Ultracel, Type C Screen, 30 kDa) of Millipore was selected as a UF/DF1 membrane. A concentration of the loading sample was 5 g/L or less in the UF, and then diafiltration was performed with 6 times or more volume of a buffer containing 50 mM Tris-HCl and 5 mM EDTA at pH 9.0. A feed flow rate was 300 LMH **or less and a transmembrane pressure (TMP) was 10 psi to 20 psi. A recommended** loading amount is 70 L/m² or less.

### (3) Anion Exchange Chromatography (AEX)

Poros 50HQ resin of Life Tech was used as a capture resin. This step was performed in a bind-and-elute mode. A recommended protein loading amount is 10 g/L resin to 50 g/L resin. A selected elution buffer is a buffer containing 100 mM His-HCl and 5 mM EDTA at pH 5.0. A recommended UV peak collection range is 25 mAU/mm to 75 mAU/mm.

### (4) Solvent/Detergent (S/D) Virus Inactivation

S/D virus inactivation was performed according to a common method.

### (5) Cation Exchange Chromatography (CEX)

This step was performed in a bind-and-elute mode. Capto S ImpAct resin of Cytiva (formerly GE Healthcare) was used as a CEX resin. A recommended CEX loading amount is 10 g/L resin to 15 g/L resin. By performing CEX, protein aggregates, host cell proteins (HCPs), and other impurities were removed. A buffer containing 50 mM Tris-HCl, 100 mM NaCl, and 5 mM EDTA at pH 8.0 is recommended as a washing buffer II, and a buffer containing 50 mM NaAc, 350 mM NaCl, and 5 mM EDTA at pH 5.5 is recommended as a washing buffer III. A recommended elution buffer is a buffer containing 50 mM Tris-HCl, 370 mM NaCl, and 5 mM EDTA at pH 8.0. A recommended UV peak collection range is 25 mAU/mm to 50 mAU/mm.

### (6) Mixed-mode Chromatography (MMC)

This step was performed in a bind-and-elute mode. Capto adhere resin of Cytiva (formerly GE Healthcare) was used as an MMC resin. A recommended loading amount is 10 g/L resin to 15 g/L resin. To further remove protein aggregates and HCPs, a buffer containing 50 mM Tris-HCl, 0.5 M arginine(Arg), and 5 mM EDTA at pH 8.0 was used as an elution buffer.

### (7) Hydrophobic interaction Chromatography (HIC)

This step was performed in a bind-and-elute mode. Butyl Sepharose 4 Fast Flow resin of Cytiva (formerly GE Healthcare) was used as an HIC resin. A recommended loading amount is 3 g/L resin to 6 g/L resin. A buffer containing 50 mM Tris-HCl and 1.5 M NaCl at pH 8.0 is recommended as a washing buffer III. The target protein was eluted with high purity using a buffer containing 50 mM Tris-HCl and 0.5 M NaCl at pH 8.0.

### (8) Ultrafiltration/Diafiltration 2 (UF/DF2)

Pellicon 3 (Ultracel, Type C Screen, 10 kDa) of Millipore was selected for UF/DF2. The loading sample was concentrated to 1 g/L to 3 g/L in UF, and subjected to diafiltration with 6 times or more volume of a buffer containing 20 mM NaAc at pH 5.0. A UF/DF2 pool concentration was 4.5 mg/mL to 5.5 mg/mL. A feed flow rate was 300 LMH or less, and a transmembrane pressure (TMP) was 10 psi to 20 psi. A load capacity is 70 g/m² or less.

### (9) Intermediate Depth Filtration (Int. DF)

For intermediate depth filtration, X0SP depth filter of Millipore was selected to remove HCPs. A recommended loading amount of the X0SP filter is 400 g/m² to 800 g/m².

### (10) Formulation and Bulk Fill

A concentration of a drug substance (DS) is 2.0±0.2 g/L. A composition of a formulation buffer was determined by drug product development (DPD) and transferred to downstream process development (DSPD). PS80 and sucrose were added to VF pool samples at final concentrations of 0.04% (w/v) and 8% (w/v), respectively. DS was obtained after 0.2 µm final filtration.

### Experimental Example 3 (not according to the invention): AEX Conditions for Specifically Isolating Specific Recombinant ECM Protein

Among ECM proteins, HAPLN1 protein has a molecular weight of 40 kDa to 50 kDa.

In the anion exchange chromatography (AEX) of Example 2-(3), an experiment was performed to optimize AEX conditions for specifically isolating a recombinant human HAPLN1 protein.

### (1) Elution conditions

### (1.1) AEX linear gradient comparative elution

### Materials:

- Column: Poros 50HQ, 3.024 mL (0.5 cm × 15.4 cm)
- Loading material: concentration 2.64 g/L, pH9.06, conductivity 1.44 mS/cm
- Loading amount: 30 g/L resin
- Column washing (sanitization) solution: 1.0 M NaOH
- Pre-equilibration buffer: 50 mM Tris-HCl, 1 M (NH₄)₂SO₄, 5 mM EDTA, pH 8.0
- Equilibration/washing buffer I: 50 mM Tris-HCl, 50 mM NaCl, 5 mM EDTA, pH 9.0
- Washing buffer II: 50 mM Tris-HCl, 10 mM NaCl, 5 mM EDTA, pH 9.0
- Elution buffer: control: 50 mM Tris-HCl, 80mM (NH₄)₂SO₄, 5mM EDTA, pH 8.5; condition 1-A: 20mM His-HCl, pH 7.5, B: 50 mM His-HCl, 30 mM NaCl, 5 mM EDTA, pH 5.8; condition 2-A: 100 mM His-HCl, pH 7.0, B: 100 mM His-HCl, 5 mM EDTA, pH 5.5; condition 3-A: 100 mM His-HCl, pH 7. 0, B: 100 mM His-HCl, 5 mM EDTA, pH 5. 0; linear gradient elution from A to B for 20 column volumes (CV)
- Stripping buffer: 50 mM Tris-HCl, 1 M (NH₄)₂SO₄, 5 mM EDTA, pH 8.0
- Column storage solution: 20% ethanol

### Experimental procedure:

Eluates were collected at 25 mAU/mm to 25 mAU/mm. The elution conditions were verified using a chromatogram.

### Results:

A yield of the recombinant human HAPLN1 protein, SEC analysis results, and HCP concentrations according to each elution condition are shown in Table 4 below. As shown in Table 4, when 100 mM His-HCl was added without salts to the elution buffer, better effect of capturing the recombinant human HAPLN1 protein was observed, as compared with those with salts. In addition, when 100 mM His-HCl was added, HCPs were decreased by 50%, as compared with a control.

**[Table 4]**

| Run | Washing buffer II | Elution conditions (linear gradient elution from A to B) | | Yield* (%) | SEC(%) M/H/L | HCP (ppm) |
|---|---|---|---|---|---|---|
| | | A | B | | | |
| 1 (Control) | - | - | 50 mM Tris-HCl, 80 mM (NH₄)₂SO₄, 5 mM EDTA, pH8.5 | 79 | 53.1/46.7/0.2 | 164608 |
| 2 (Elut ion condition 1) | 50 mM Tris-HCl, 10 mM NaCl, 5 mM EDTA, pH9.0 | 20 mM His-HCl, pH7.5 100 mM His-HCl, pH7.0 | 50 mM His-HCl, 30 mM NaCl, 5 mM EDTA, pH5.8 | 74 | 56.7/43.3/ND | 73112 |
| 3 (Elution condition 2) | | | 100 mM His-HCl, 5 mM EDTA, pH5.5 | 78 | 55.8/44.2/ND | 79856 |
| 4 (Elution condition 3) | | | 100 mM His-HCl, 5 mM EDTA, pH5.0 | 85 | 54.4/45.6/ND | 83654 |

### (1.2) AEX linear gradient elution

### Materials:

- Column: Poros 50HQ, 3.024 mL (0.5 cm × 15.4 cm)
- Loading material: concentration 2.64 mg/mL, pH 9.06, conductivity 1.44 mS/cm
- Loading amount: 30 g/L resin
- Sanitization solution: 1.0 M NaOH
- Pre-equilibration buffer: 50 mM Tris-HCl , 1 M (NH₄)₂SO₄, 5 mM EDTA, pH 8.0
- Equilibration/washing buffer I: 50 mM Tris-HCl, 50 mM NaCl, 5 mM EDTA, pH 9.0
- Washing buffer II: 50 mM Tris-HCl, 5 mM EDTA, pH 9.0
- Elution buffer: A: 100 mM His-HCl, pH 7.0, B: 100 mM His-HCl, 5 mM EDTA, pH 5.0; linear gradient elution from A to B for 20 column volumes (CV).
- Stripping buffer: 50 mM Tris-HCl, 450 mM (NH₄)₂SO₄, 5 mM EDTA, pH 8.0
- Storage solution: 20% ethanol

### Experimental procedure:

Based on the results of linear gradient comparative elution, the material after UF/DF1 was loaded into a Poros 50HQ column, and an optimal elution buffer was determined using a step-wise elution method. Optimal elution conditions were confirmed using SEC purity analysis.

### Results:

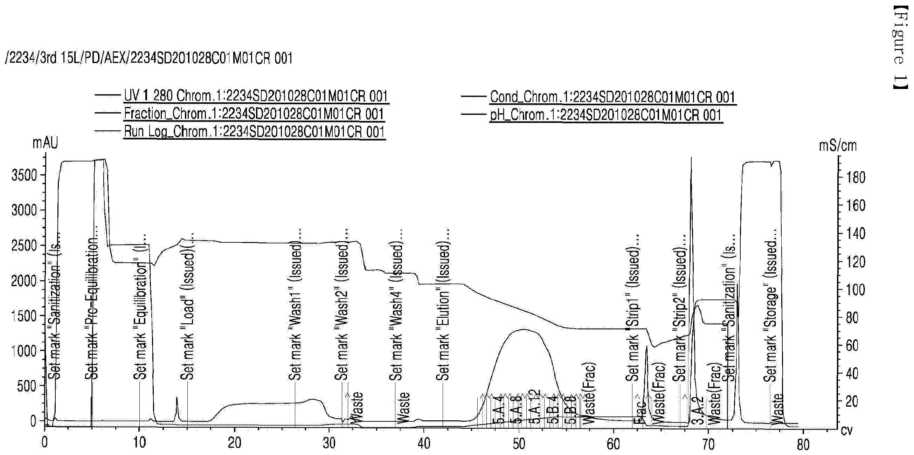

FIG. 1 shows a chromatogram of the linear gradient elution of AEX.

As shown in Table 4 and FIG. 1, 100 mM His-HCl efficiently captured the recombinant human HAPLN1 protein without additional salt addition. For a balance between purity and yield, pH 5.0 was chosen as pH for elution.

Therefore, it was found that about 100 mM His-HCl may be selected as the AEX elution buffer in order to specifically isolate the recombinant human HAPLN1 protein. In particular, when 100 mM His-HCl is used, the recombinant human HAPLN1 protein may be isolated with excellent purity and yield. For example, a buffer containing 100 mM His-HCl and 5 mM EDTA at pH 5.0 may be used as the AEX elution buffer.

### Experimental Example 4 (not according to the invention): CEX conditions for removing recombinant ECM protein aggregates, HCPs, and other impurities

The bind-and-elute mode CEX of Example 2-(5) was introduced to remove recombinant human HAPLN1 protein aggregates, HCPs, and other impurities. Therefore, an experiment was performed to optimize the CEX conditions for removing recombinant human HAPLN1 protein aggregates, HCPs, and other impurities.

### (1) Elution conditions

### Materials:

- Column: Capto S ImpAct, 2.631 mL (0.5 cm × 13.4 cm)
- Loading material: AEX eluate, concentration 11.844 mg/mL, pH 5.52, conductivity 11.70 mS/cm
- Loading amount: 10 mg/mL resin
- Equilibration/Washing I buffer: 50 mM NaAc-HAc, 5 mM EDTA, pH 5.5
- Washing II buffer: 50 mM Tris-HCl, 100 mM NaCl, 5 mM EDTA, pH 8.0
- Washing III buffer: 50 mM NaAC, 350 mM NaCl, 5 mM EDTA, pH 5.5
- Elution buffer: (A) 50 mM Tris-HCl, 5 mM EDTA, pH 8.0; (B) 50 mM Tris-HCl, 500 mM NaCl, 5 mM EDTA, pH 8.0; step-wise elution: 20% B (100 mM NaCl), 5 CV; 40% B (200 mM NaCl), 5 CV: 60% B (300 mM NaCl), 5 CV; 75% B (375 mM NaCl), 5 CV: 85% B (425 mM NaCl)
- Stripping buffer: 50 mM Tris-HCl, 500 mM NaCl, 5 mM EDTA, pH 8.0

### Experimental procedure:

Optimal elution conditions were confirmed by performing a step-wise elution method. A loading amount was 10 g/L resin, and eluates were collected at 25 mAU/mm to 25 mAU/mm. A protein concentration of each fraction was measured, and step-wise recovery amounts were calculated. In addition, sample purity was analyzed by SDS_PAGE_NR.

### Results:

The elution conditions are important for product quality. Criteria for the optimal conditions are based on the removal of impurities.

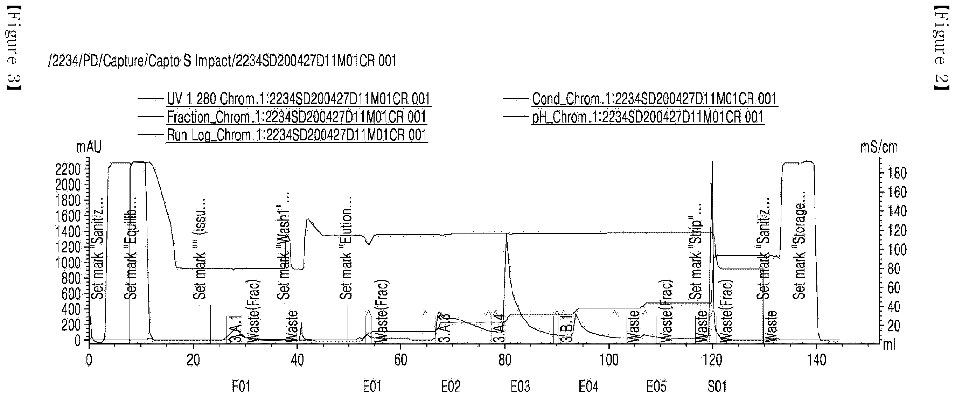

FIG. 2 shows a chromatogram of the step-wise elution of CEX.

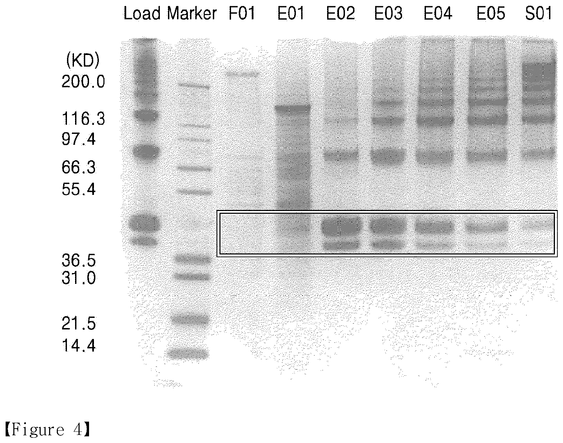

FIG. 3 shows a result of SDS_PAGE_NR of the step-wise elution of CEX.

The HCP test results in the CEX step-wise elution are shown in Table 5 below.

**[Table 5]**

| Fract ion | NaCl concentrat ion (mM) | yield (%) | HCP (ng/mg) |
|---|---|---|---|
| Load | - | - | 121669 |
| F01 | 0 | 2.0 | - |
| E01 | 100 | 0.7 | 799110 |
| E02 | 200 | 20.0 | 115447 |
| E03 | 300 | 32.0 | |
| E04 | 375 | 9.8 | 35248 |
| E05 | 425 | 1.6 | - |
| S01 | 500 | 9.2 | 11179 |

As shown in FIG. 3 and Table 5, when 100 mM NaCl (E01) was used, the largest amount of HCPs was removed, and loss of the target protein was only 0.7%. Yields of fraction E02-E04 and fraction E05 were 61.8% and 1.6%, respectively. The HMW content was increased with increasing NaCl. For a balance between purity and yield of the product, a buffer containing 50 mM Tris-HCl, 370 mM NaCl, 5 mM EDTA at pH 8.0 was recommended as the elution buffer.

### Experimental Example 5 (not according to the invention): MMC conditions for removing recombinant ECM protein aggregates and HCPs

The bind-and-elute mode MMC of Example 2-(6) was used to additionally remove recombinant human HAPLN1 protein aggregates and HCPs. Therefore, an experiment was performed to optimize the MMC conditions for removing recombinant human HAPLN1 protein aggregates and HCPs.

### (1) Elution conditions

The recombinant human HAPLN1 protein aggregates and HCPs were removed using a capto adhere. CEX eluates were loaded onto the Capto adhere column. Based on the result of linear gradient elution for 50 L material production, optimal elution conditions were confirmed by a step-wise elution method.

### Materials:

- Column: Capto adhere, 2.985 mL (0.5 cm × 15.2 cm)
- Loading material: CEX eluate, concentration 3.105 mg/mL, HCP 155217 ng/mg, SEC purity 52.9%
- Loading amount: 7.5 g/L resin
- Pre-equilibration buffer: 50 mM NaAc-HAc, 1 M NaCl, 5 mM EDTA, pH 5.5
- Equilibration/washing buffer I: 50 mM Tris-HCl, 5 mM EDTA, pH 8.0
- Elution buffer: (A) 50 mM Tris-HCl, 5 mM EDTA, pH 8.0; (B) 50 mM Tris-HCl, 1 M Arg, 5 mM EDTA, pH 8.0; step-wise elution: 20% B (200 mM Arg), 5 CV; 40% B (400 mM Arg), 10 CV; 50% B (500 mM Arg), 10 CV; 60% B (600 mM Arg), 10 CV; 70% B (700 mM Arg), 10 CV
- Stripping buffer: 50 mM HAc

### Experimental procedure:

The optimal elution conditions were determined by step-wise elution. CEX eluates were loaded into the Capto adhere column with 7.5 g/L resin, and the eluates were collected at 25 mAU/mm to 25 mAU/mm. A protein concentration of each fraction was measured, and step-wise recovery amounts were calculated. HCP and SDS_PAGE_NR purity were also tested.

### Results:

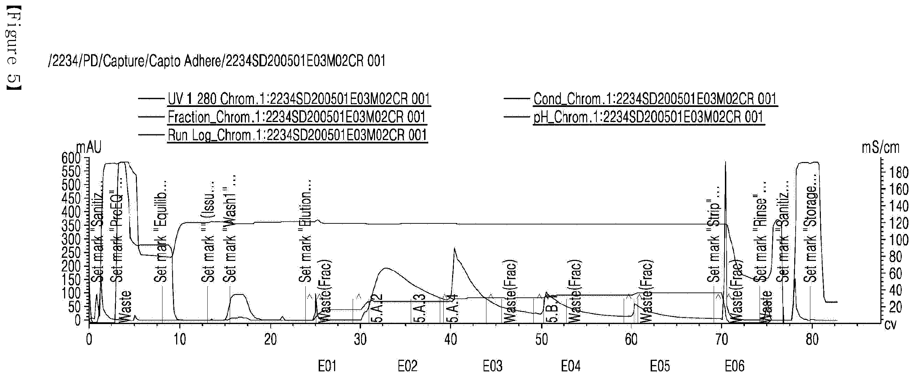

FIG. 4 shows a chromatogram of the step-wise elution of MMC.

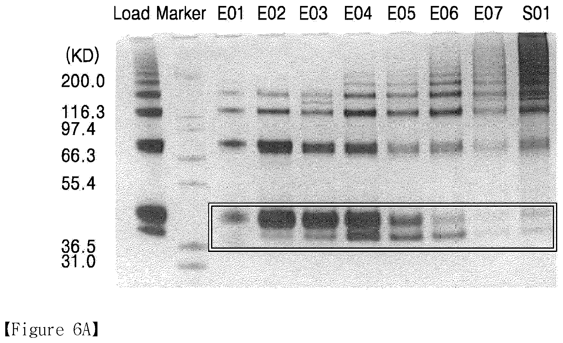

FIG. 5 shows a result of SDS_PAGE_NR of the step-wise elution of MMC.

The results of testing HCPs in the MMC step-wise elution are shown in Table 6 below.

**[Table 6]**

| Fraction | Arginine concentration (mM) | Yield (%) | HCP (ng/mg) |
|---|---|---|---|
| Load | - | - | 155217 |
| Peak 1 (E01) | 200 | 0.2 | 2711333 |
| Peak 2 (E02-E03) | 400 | 39.5 | 39995 |
| Peak 3 (E04-E05) | 500 | 28.1 | 4295 |
| Peak 4 (E06) | 600 | 6.6 | 5865 |
| Peak 5 (E07) | 700 | 0.7 | - |
| Peak 6 (S01) | 1000 | 1.9 | 27198 |

As shown in FIG. 5 and Table 6, when 200 mM arginine (peak 1) was used, the largest amount of HCPs was removed, and loss of the target protein was only 0.2%. Yields of peak 2 and peak 3 were 39.5% and 28.1%, respectively. When the arginine concentration was high, the largest amount of HMW was eluted. For a balance between purity and yield of the product, a buffer containing 50 mM Tris-HCl, 500 mM arginine, and 5 mM EDTA at pH 8.0 was recommended as the elution buffer. 200 mM arginine may be used in washing.

### Experimental Example 6 (not according to the invention): HIC Conditions for Improving Purity of Recombinant ECM Protein

The HIC of Example 2-(7) was used to remove recombinant human HAPLN1 protein multimers and HCPs. Therefore, an experiment was performed to optimize the HIC conditions for removing the recombinant human HAPLN1 protein multimers and HCPs.

### (1) Elution conditions

### Materials:

- Column: Butyl Sepharose 4 Fast Flow, 2.631 mL (0.5 cm × 13.4 cm)
- Loading materials: 1) MMC eluate, concentration 0.590 mg/mL, pH 8.09, 146.35 mS/cm, SEC purity 68.9%; 2) MMC eluate, concentration 0.543 mg/mL, pH 8.10, 145.82 mS/cm, SEC purity 68.9%
- Loading amount: 5 g/L resin
- Equilibration/washing buffer I: 50 mM Tris-HCl, 1 M (NH₄)₂SO₄, 5 mM EDTA, pH 8.0
- Washing buffer II: 50 mM Tris-HCl, 0.4 M (NH₄)₂SO₄, 5 mM EDTA, pH 8.0
- Washing buffer III: 1) 50 mM Tris-HCl, 2 M NaCl, 5 mM EDTA, pH 8.0; 2) 50 mM Tris-HCl, 1.5 M NaCl, 5 mM EDTA, pH 8.0
- Elution buffer:
   1) (A) 50 mM Tris-HCl, 2 M NaCl, 5 mM EDTA, pH 8.0; (B) 50 mM Tris-HCl, 5 mM EDTA, pH 8.0; step-wise elution: 25% B (1.5 M NaCl), 10 CV; 50% B (1 M NaCl), 10 CV; 75% B (0.5 M NaCl), 10 CV; 90% B (0.2 M NaCl), 10 CV; 100% B (0 M NaCl), 10 CV:
   2) 50 mM Tris-HCl, 0.5 M NaCl, pH 8.0
- Stripping buffer: 50 mM Tris-HCl, 5 mM EDTA, pH 8.0

### Experimental procedure:

Washing III and elution conditions were determined by performing a step-wise elution method. MMC eluates were adjusted with ~1 M (NH₄)₂SO₄ before loading on the HIC column. A loading amount was 5 g/L resin, and eluates were collected at 25 mAU/mm to 25 mAU/mm. A protein concentration of each fraction was measured, and step-wise recovery amounts were calculated. Purity was tested using SDS_PAGE_NR.

### Results:

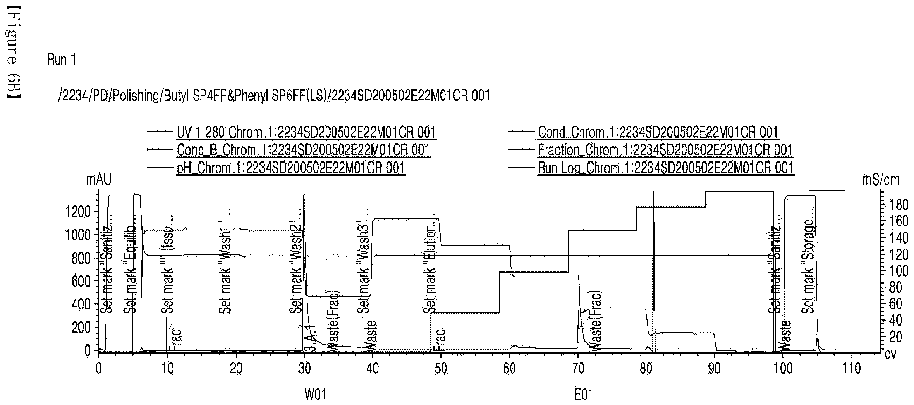

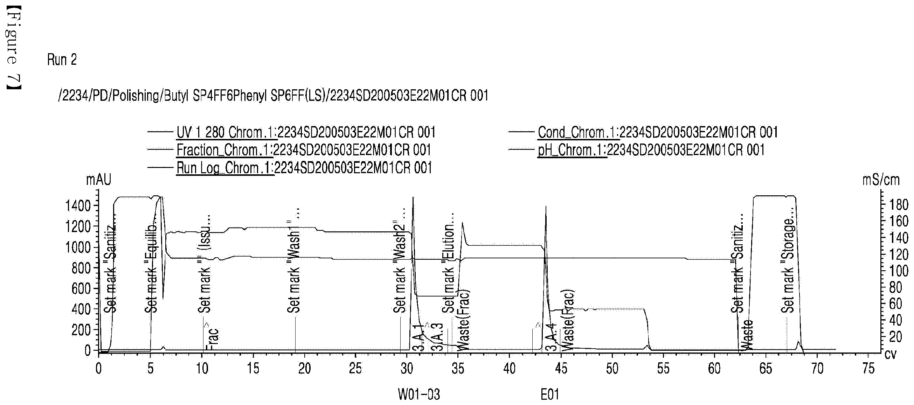

FIGS. 6A and 6B show a chromatogram of HIC comparative elution.

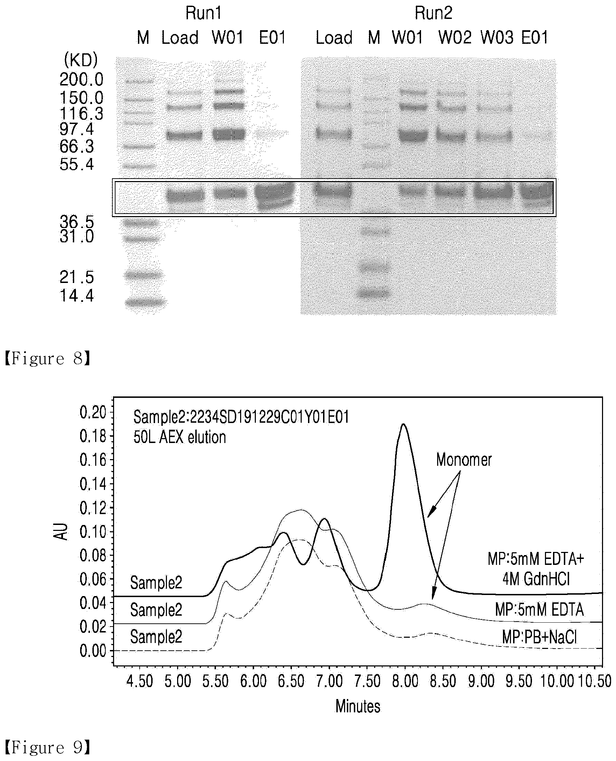

FIG. 7 shows a result of SDS_PAGE_NR of HIC comparative elution.

The results of the HIC step-wise elution are shown in Table 7 below.

**[Table 7]**

| Run No. | Washing III conditions | Elution conditions | Fraction | Yield (%) | SEC purity (%) |
|---|---|---|---|---|---|
| 1) | 2 M NaCl | Step-wise (1.5 → 1 → 0.5 → 0.2 → 0 M NaCl) | Washing | 45.8 | - |
| | | | Elution | 15.3 | - |
| 2) | 1.5 M NaCl | One-step (0.5 M NaCl) | Washing | 50.1 | - |
| | | | Elution | 34.8 | 96.7/3.3/ND |

As shown in FIG. 7 and Table 7, the largest amount of HMW was removed by the washing buffer II, and the target protein was not eluted in the presence of NaCl exceeding 1.5 M at pH 8.0. Therefore, in Run2, a buffer containing 50 mM Tris-HCl, 1.5 M NaCl, and 5 mM EDTA at pH 8.0 was used in Wash III, and a buffer containing 50 mM Tris-HCl, 0.5 M NaCl, and 5 mM EDTA at pH 8.0 was used in elution. Finally, a yield of the second experiment was 34.8%. Therefore, a buffer containing 50 mM Tris-HCl, 1.5 M NaCl, and 5 mM EDTA at pH 8.0 was used as the washing buffer III, and a buffer containing 50 mM Tris-HCl and 0.5 M NaCl at pH 8.0 was used as the elution buffer.

### Example 3: Method of Assaying Recombinant ECM Proteins

The monomers of the recombinant ECM protein and other impurities in samples were analyzed by performing size exclusion chromatography (SEC) using a mobile phase containing hydrochloride. Specific conditions for SEC are as follows:
- Column: TSKgel G3000SWXL, 7.8 × 300 mm, 5 µm Steel (Manuf. TOSOH)
- Mobile phase: 50 mM phosphate buffer (PB), 300 mM NaCl, 1 M Gdn-HCl or Arg-HCl pH7.5 (±0.5)
- Detection wavelength: 280 nm
- Flow rate: 1.0 mL/min
- Column temperature: 25 ± 3 °C
- Sample temperature: 5 ± 3°C
- Sample feeding amount: 100 µg

### Experimental Example 7: Screening for Additives for Accurate Analysis of Recombinant ECM Proteins

An experiment was performed to screen for additives of a mobile phase, which is able to improve accuracy by reducing appearance of inaccurate peaks, when the monomers of the recombinant ECM protein and other impurities are analyzed using SEC. In detail, to analyze the monomers of the recombinant human HAPLN1 protein and other impurities using SEC, accuracy of SEC analysis according to the type and concentration of the additive used as the mobile phase was examined. As a sample, an intermediate product during the isolation and purification of the recombinant human HAPLN1 protein according to Example 2 was used.

FIG. 8 shows a chromatogram showing a result of performing SEC analysis for a sample including the recombinant human HAPLN1 protein using phosphate buffer(PB)+NaCl, 5 mM EDTA, or 5 mM EDTA+4 M Gdn-HCl as the mobile phase (MP).

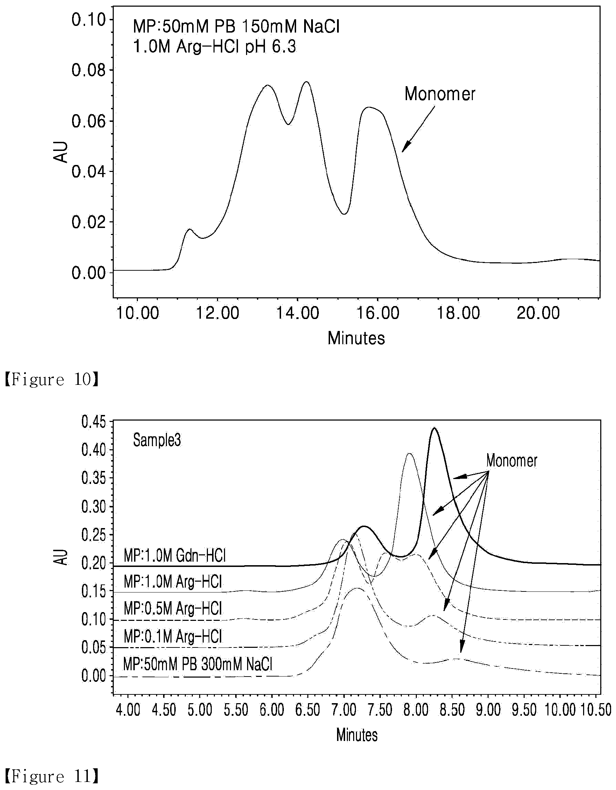

FIG. 9 shows a chromatogram showing a result of performing SEC analysis for a sample including the recombinant human HAPLN1 protein using 50 mM PB+150 mM NaCl+1M Arg-HCl (pH 6.3) as the mobile phase.

As shown in FIGS. 8 to 9, when hydrochloride was added to the mobile phase, the monomer peak appeared most clearly.

FIG. 10 shows a chromatogram showing a result of performing SEC analysis using 50 mM PB+300 mM NaCl, 0.1 M Arg-HCl, 0.5 M Arg-HCl, 1.0 M Arg-HCl, or 1.0 M Gdn-HCl as the mobile phase. The analysis results are shown in Table 8 below.

**[Table 8]**

| Mobile phase | Monomer (%) | HMW (%) | LMW (%) |
|---|---|---|---|
| 50 mM PB+300 mM NaCl | 22.4 | 76.9 | 0.8 |
| 0.1 M Arg-HCl | 26.1 | 73.9 | ND |
| 0.5 M Arg-HCl | 62.0 | 38.0 | ND |
| 1.0 M Arg-HCl | 71.8 | 28.2 | ND |
| 1.0 M Gdn-HCl | 77.2 | 22.8 | ND |

As shown in FIG. 10 and Table 8, the recombinant human HAPLN1 protein was well isolated with increasing concentration of Arg-HCl. In particular, when Arg-HCl and Gdn-HCl were used at a concentration of 1.0 M, the ability to isolate the recombinant human HAPLN1 protein was excellent.

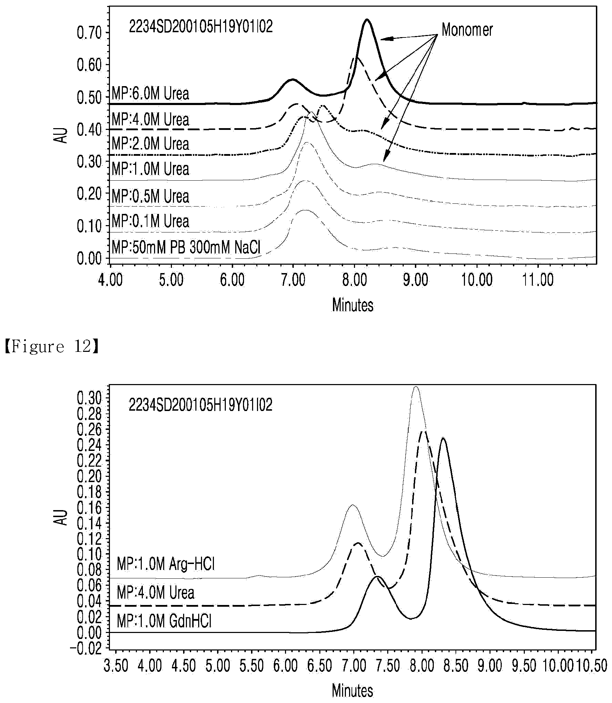

FIG. 11 shows a chromatogram showing a result of performing SEC analysis using urea, which is known as a monomer assay additive, as a mobile phase, such as 50 mM PB+300 mM NaCl, 0.1 M urea, 0.5 M urea, 1.0 M urea, 2.0 M urea, 4.0 M urea, or 6.0 M urea. The analysis results are shown in Table 9 below.

**[Table 9]**

| Mobile phase | Monomer (%) | HMW (%) | LMW (%) |
|---|---|---|---|
| 50 mM PB+300 mM NaCl | 26.4 | 73.6 | ND |
| 0.1 M urea | 26.4 | 73.6 | ND |
| 0.5 M urea | 26.7 | 73.3 | ND |
| 1.0 M urea | 27.7 | 72.3 | ND |
| 2.0 M urea | 32.1 | 67.9 | ND |
| 4.0 M urea | 75.6 | 24.4 | ND |
| 6.0 M urea | 75.6 | 24.4 | ND |

As shown in FIG. 11 and Table 9, the recombinant human HAPLN1 protein was well isolated with increasing concentration of urea. However, unlike hydrochloride, when urea was used at a high concentration of 4.0 M or more, it was possible to isolate the recombinant human HAPLN1 protein.

FIG. 12 shows a chromatogram showing a result of performing SEC analysis using 1.0 M Gdn-HCl, 4.0 M urea, or 1.0 M Arg-HCl as a mobile phase. The analysis results are shown in Table 10 below.

**[Table 10]**

| Mobile phase | Monomer (%) | HMW (%) | LMW (%) |
|---|---|---|---|
| 1.0 M Gdn-HCl | 77.2 | 22.8 | ND |
| 4.0 M urea | 75.6 | 24.4 | ND |
| 1.0 M Arg-HCl | 71.8 | 28.2 | ND |

As shown in FIG. 12 and Table 10, when hydrochloride such as Gdn-HCl and Arg-HCl is used even at a low concentration of 1.0 M, the ability to isolate the recombinant human HAPLN1 protein was equivalent to or higher than that of using urea at a high concentration of 4.0 M.

Therefore, when SEC analysis was performed using 1.0 M hydrochloride as the additive of the mobile phase, the excellent ability to isolate the recombinant human HAPLN1 protein was observed, and inaccurate peaks in the chromatogram were significantly reduced, allowing accurate analysis of the monomer of the recombinant human HAPLN1 protein.

Taken together, it was confirmed that a ratio of the recombinant ECM protein monomer in the sample may be analyzed by performing size exclusion chromatography using the mobile phase containing hydrochloride.

## Claims

1. A method of assaying a monomer of a recombinant extracellular matrix protein, the method comprising:
performing size exclusion chromatography on a sample comprising the recombinant extracellular matrix protein using a mobile phase comprising hydrochloride at a concentration of 0.5 M to 1.2 M; and
analyzing the monomer of the recombinant extracellular matrix protein in the sample, based on a result of the size exclusion chromatography;
wherein the recombinant extracellular matrix protein is elastin, fibronectin, laminin, vitronectin, tenascin, or hyaluronan and proteoglycan link protein (HAPLN).

2. The method of claim 1, wherein the recombinant HAPLN protein is any one protein selected from the group consisting of HAPLN1, HAPLN2, HAPLN3, and HAPLN4.

3. The method of claim 1, wherein the hydrochloride is arginine hydrochloride, aniline hydrochloride, adenine hydrochloride, guanine hydrochloride, guanidine hydrochloride, histidine hydrochloride, or lysine hydrochloride.

4. The method of claim 1, wherein, in the analyzing, ratios of the monomer of the recombinant extracellular matrix protein and other impurities are analyzed.

## Patentansprüche

1. Verfahren zum Untersuchen eines Monomers eines rekombinanten Proteins einer extrazellulären Matrix, wobei das Verfahren Folgendes umfasst:
Durchführen einer Größenausschlusschromatographie auf einer Probe, umfassend das rekombinante Protein einer extrazellulären Matrix, unter Verwendung einer mobilen Phase, umfassend Hydrochlorid in einer Konzentration von 0,5 M bis 1,2 M; und
Analysieren des Monomers des rekombinanten Proteins einer extrazellulären Matrix in der Probe, basierend auf dem Ergebnis der Größenausschlusschromatographie;
wobei das rekombinante Protein einer extrazellulären Matrix Elastin, Fibronectin, Laminin, Vitronectin, Tenascin oder Hyaluronan- und Proteoglycan-Verbindungsprotein (HAPLN) ist.

2. Verfahren nach Anspruch 1, wobei das rekombinante HAPLN-Protein ein beliebiges Protein ist, das aus der aus HAPLN1, HAPLN2, HAPLN3 und HAPLN4 bestehenden Gruppe ausgewählt ist.

3. Verfahren nach Anspruch 1, wobei das Hydrochlorid Argininhydrochlorid, Anilinhydrochlorid, Adeninhydrochlorid, Guaninhydrochlorid, Guanidinhydrochlorid, Histidinhydrochlorid oder Lysinhydrochlorid ist.

4. Verfahren nach Anspruch 1, wobei bei der Analyse Anteile des Monomers des rekombinanten Proteins einer extrazellulären Matrix und anderer Verunreinigungen analysiert werden.

## Revendications

1. Procédé de dosage d'un monomère d'une protéine de matrice extracellulaire recombinante, le procédé comprenant les étapes consistant à :
effectuer une chromatographie d'exclusion de taille sur un échantillon comprenant la protéine de matrice extracellulaire recombinante en utilisant une phase mobile comprenant du chlorhydrate à une concentration de 0,5 M à 1,2 M ; et
analyser le monomère de la protéine de matrice extracellulaire recombinante dans l'échantillon, sur la base d'un résultat de la chromatographie d'exclusion de taille ;
dans lequel la protéine de matrice extracellulaire recombinante est l'élastine, la fibronectine, la laminine, la vitronectine, la ténascine ou et la protéine de liaison de hyaluronane et protéoglycane (HAPLN).

2. Procédé selon la revendication 1, dans lequel la protéine HAPLN recombinante est n'importe quelle protéine choisie dans le groupe constitué de HAPLN1, HAPLN2, HAPLN3, et HAPLN4.

3. Procédé selon la revendication 1, dans lequel le chlorhydrate est du chlorhydrate d'arginine, du chlorhydrate d'aniline, du chlorhydrate d'adénine, du chlorhydrate de guanine, du chlorhydrate de guanidine, du chlorhydrate d'histidine ou du chlorhydrate de lysine.

4. Procédé selon la revendication 1, dans lequel, dans l'analyse, les rapports du monomère de la protéine de matrice extracellulaire recombinante et d'autres impuretés sont analysés.
